# EUROPEAN PATENT APPLICATION

(11) **EP 3 939 632 A1**
(43) Date of publication of application: **19.01.2022**
(21) Application number: 20770355.4
(22) Date of filing: 07.02.2020
(51) Int. Cl.: A61M 1/02, A61J 1/10

(54) **BLOOD BAG SYSTEM**

(30) Priority: 14.03.2019 JP 2019047051
(71) Applicant: TERUMO Kabushiki Kaisha, Tokyo 151-0072 (JP)
(72) Inventor: AGEISHI, Aya, Tokyo 163-1450 (JP); MARUTA, Chiaki, Tokyo 163-1450 (JP)
(74) Representative: TBK
(86) International application number: PCT/JP2020/004794
(87) International publication number: WO 2020/184018

(57) **Abstract**

A blood bag system (10) is provided with a liquid drug bag (26) having an internal storage space (26a) in which a liquid drug is stored, and a third tube (38) that is connected to the liquid drug bag (26) and has a flow path (38a) therein connected to the storage space (26a). A retention mechanism (98) for separably retaining the liquid drug bag (26) and the third tube (38) is provided between the outer surface of the liquid drug bag (26) and the outer peripheral surface of the third tube (38). The third tube (38) can thus be inserted together with the liquid drug bag (26) when the liquid drug bag (26) is accommodated in a liquid drug bag pocket (60) of a centrifugal transport device (12).

## Description

### Technical Field

The present invention relates to a blood bag system set in a centrifugal transport device.

### Background Art

A blood bag system including a plurality of bags (a blood bag storing blood, a liquid drug bag storing a liquid drug, and the like) is set in a centrifugal transport device at the time of centrifuging blood (see JP H9-168585 A). The centrifugal transport device applies a centrifugal force to the set blood bag to separate the blood in the blood bag into a plurality of types of blood components, and transports a predetermined blood component to another bag via a tube connected to the blood bag. In addition, the liquid drug in the liquid drug bag is transported to a bag storing a predetermined blood component after the blood bag system is taken out from the centrifugal transport device.

### Summary of Invention

Meanwhile, the liquid drug bag of the blood bag system is accommodated in a liquid drug bag accommodating portion (liquid drug bag pocket) when being set in the centrifugal transport device. At this time, the tube connected to the liquid drug bag is also accommodated in the liquid drug bag pocket together with the liquid drug bag. This is because there is a possibility that the tube interferes with a peripheral structure of the centrifugal transport device during centrifugation if the tube projects from the centrifugal transport device.

In the blood bag system, however, the tube connected to the liquid drug bag is long, and thus, it is difficult to accommodate the liquid drug bag and the tube together in the liquid drug bag pocket, and there is a problem that it takes time and effort for the setting work of the centrifugal transport device.

The present invention has been made to solve the above problems, and an object thereof is to provide a blood bag system capable of significantly improving work efficiency by enabling smooth accommodation of a liquid drug bag with a simple configuration.

In order to achieve the above object, an aspect of the present invention is a blood bag system including: a liquid drug bag that has an internal storage space in which a liquid drug is stored and is accommodated in a bag accommodating portion of a centrifugal transport device; and a tube that has one end connected to the liquid drug bag and has a flow path communicating with the storage space inside. A retention mechanism for separably retaining the liquid drug bag and the tube is provided between an intermediate portion, between the one end and another end of the tube, and the liquid drug bag.

The liquid drug bag and the tube can be integrally inserted when the liquid drug bag is accommodated in the bag accommodating portion of the centrifugal transport device with a simple configuration in which the blood bag system includes the retention mechanism for separably retaining the liquid drug bag and the tube. As a result, the tube is prevented from interfering with the insertion of the liquid drug bag, the liquid drug bag can be smoothly accommodated, and the work efficiency at the time of setting can be significantly improved. In addition, after the blood bag system is taken out from the centrifugal transport device, the liquid drug can be favorably supplied from the liquid drug bag suspended on a stand thereafter by separating the tube from the liquid drug bag.

### Brief Description of Drawings

Fig. 1 is an explanatory view illustrating an overall configuration of a blood bag system and a centrifugal transport device according to a first embodiment of the present invention.
Fig. 2 is a plan view illustrating a unit set area of the centrifugal transport device.
Fig. 3A is a front view illustrating a liquid drug bag. Fig. 3B is a cross-sectional view taken along line IIIB-IIIB.
Fig. 4A is an explanatory view illustrating an operation of accommodating the liquid drug bag. Fig. 4B is an explanatory view illustrating an operation of separating the liquid drug bag and a third tube.
Fig. 5 is a front view partially illustrating a liquid drug bag and a third tube of a blood bag system according to a second embodiment of the present invention.
Fig. 6A is an enlarged perspective view illustrating a retaining member in Fig. 5. Fig. 6B is an enlarged perspective view illustrating another configuration example of the retaining member.
Fig. 7 is a front view partially illustrating a liquid drug bag and a third tube of a blood bag system according to a third embodiment of the present invention.

### Description of Embodiments

Hereinafter, preferred embodiments of the present invention will be described in detail with reference to the accompanying drawings.

### [First Embodiment]

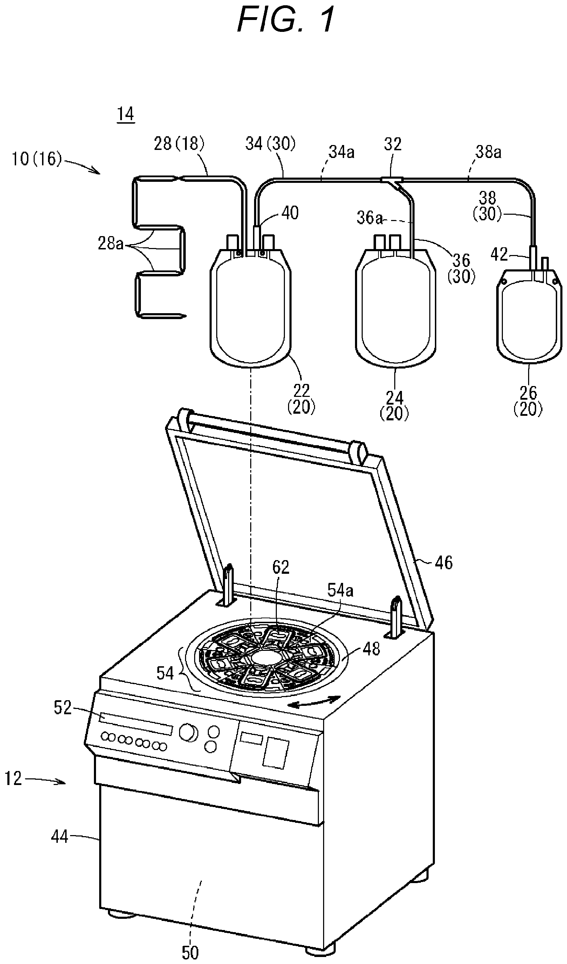

A blood bag system 10 according to a first embodiment of the present invention is set in a centrifugal transport device 12 as illustrated in Fig. 1 by a user such as a medical worker. The centrifugal transport device 12 centrifuges blood in the blood bag system 10 to generate and store a plurality of types of blood components. Hereinafter, a configuration including the blood bag system 10 and the centrifugal transport device 12 is referred to as a blood product manufacturing system 14.

The blood bag system 10 includes a pretreatment unit (not illustrated) used for blood sampling before centrifugation and a separator unit 16 that generates blood components by centrifugation and individually stores the blood components, and the both units are connected by a relay tube 18. The separator unit 16 is set in the centrifugal transport device 12 in a state where the relay tube 18 connected to the pretreatment unit is cut into the state illustrated in Fig. 1 before the centrifugation.

The pretreatment unit of the blood bag system 10 collects whole blood from a donor (blood donor) and removes a predetermined blood component (for example, a white blood cell) from the whole blood. Therefore, the pretreatment unit includes a blood collection needle, a blood collection bag, an initial flow blood bag, a filter (for example, a white blood cell removal filter), and the like, and is configured by connecting the respective members via a plurality of tubes. The separator unit 16 is connected to the downstream side of the filter via the relay tube 18. Specifically, the pretreatment unit stores the first blood out of the whole blood of the donor, collected through the blood collection needle, in the initial flow blood bag, and then stores the remaining blood in the blood collection bag. Further, the pretreatment unit removes white blood cells in the filter by causing the whole blood, stored in the blood collection bag, to flow to the filter, and transports the removed blood (white-blood-cell-removed blood) to the separator unit 16.

On the other hand, the separator unit 16 of the blood bag system 10 includes a plurality of bags 20 (a blood bag 22, a PPP bag 24, and a liquid drug bag 26), and is configured by connecting the respective bags 20 via a plurality of tubes 30.

The blood bag 22 is directly connected to the relay tube 18 connected to the pretreatment unit in a state of the blood bag system 10 provided as a product. Therefore, the blood bag 22 stores the removed blood transported from the filter at the time of collecting blood. The blood bag 22 is set in the centrifugal transport device 12, and a centrifugal force is applied by the operation of the centrifugal transport device 12. As a result, the removed blood in the blood bag 22 is centrifuged into blood components (platelet poor plasma (PPP), red blood cells (RBC)) and the like having different specific gravities. Then, the blood bag 22 transports PPP under the operation of the centrifugal transport device 12 after centrifugation to store only the remaining RBC.

The PPP bag 24 stores PPP by receiving the PPP supplied from the blood bag 22. On the other hand, the liquid drug bag 26 contains a red blood cell preservation solution (liquid drug), such as a MAP solution, an SAGM solution, and OPTISOL, in advance.

In addition, a segment tube 28 is formed before the separator unit 16 is set in the centrifugal transport device 12. The segment tube 28 has a plurality of sealed tubes 28a in which blood of the donor (removed blood) exists. The plurality of sealed tubes 28a are formed so as to be continuous in series by cutting the relay tube 18, which connects the blood bag 22 and the pretreatment unit (filter), near the filter and further sealing the cut tube at predetermined intervals. The segment tube 28 is cut by a user as necessary, and is used for checking blood or the like.

The plurality of tubes 30 of the separator unit 16 branch into a plurality of tubes via a branching connector 32 (Y-type connector). Specifically, the plurality of tubes 30 include a first tube 34 connecting the blood bag 22 and the branching connector 32, a second tube 36 connecting the PPP bag 24 and the branching connector 32, and a third tube 38 connecting the liquid drug bag 26 and the branching connector 32. The first tube 34 has a first flow path 34a, the second tube 36 has a second flow path 36a, and the third tube 38 has a third flow path 38a. The first to third flow paths 34a, 36a, and 38a communicate with each other via a flow path in the branching connector 32.

A breakable sealing member 40 (click tip) is provided at an end portion of the first tube 34 on the blood bag 22 side. Similarly, a breakable sealing member 42 is provided at an end portion of the third tube 38 on the liquid drug bag 26 side. The sealing members 40 and 42 block the first flow path 34a and the third flow path 38a until a breaking operation is performed, and prevent a liquid in the blood bag 22 and the liquid drug bag 26 from being transported to the other bag 20.

On the other hand, the centrifugal transport device 12 in which the separator unit 16 of the blood bag system 10 is set includes a box-shaped base body 44, a lid 46 capable of opening and closing an upper surface of the base body 44, and a centrifugal drum 48 provided on the base body 44. In addition, the base body 44 of the centrifugal transport device 12 is provided with a motor (not illustrated) that rotates the centrifugal drum 48, a control unit 50 that controls the operation of the centrifugal transport device 12, and an operation display unit 52 configured to allow the user to perform confirmation and operation.

The centrifugal drum 48 has a plurality of (six) unit set areas 54 in which the separator unit 16 can be set. A height of one unit set area 54 is longer than a longitudinal length of the bag 20, and is set in a range of 60° with respect to a rotation center of the centrifugal drum 48. That is, the six unit set areas 54 are arrayed without a gap along the circumferential direction to form the centrifugal drum 48 which is an annular structure.

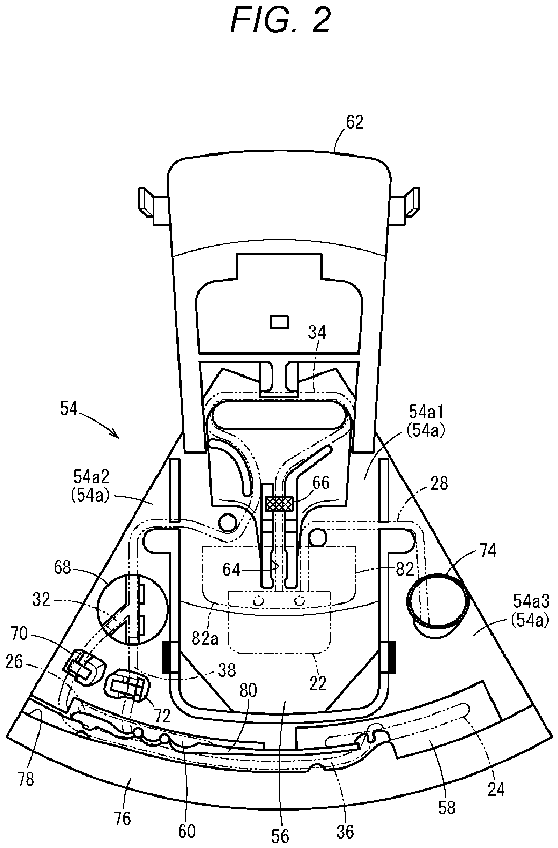

As illustrated in Figs. 1 and 2, the unit set area 54 applies the centrifugal force to the blood bag 22 as the centrifugal drum 48 rotates. Specifically, the unit set area 54 includes a blood bag pocket 56 accommodating the blood bag 22, a PPP bag pocket 58 accommodating a PPP bag 24, and a liquid drug bag pocket 60 (bag accommodating portion) accommodating the liquid drug bag 26 at radially outer positions of the centrifugal drum 48.

The blood bag pocket 56 is provided at the circumferential center of the unit set area 54 and has a larger volume than the PPP bag pocket 58 or the liquid drug bag pocket 60. The PPP bag pocket 58 and the liquid drug bag pocket 60 are provided side by side in the circumferential direction on the radially outer side of the blood bag pocket 56.

In addition, an upper surface 54a of the unit set area 54 is configured such that the plurality of tubes 30 of the blood bag system 10 are disposed and retained thereon. The first tube 34 and a part of the segment tube 28 are disposed in a central region 54a1 (first region) on the radially inner side of the blood bag pocket 56 on the upper surface 54a. The central region 54a1 is provided with a lid body 62 that opens and closes an opening of the blood bag pocket 56. Further, a sensor 66 (optical sensor), which detects a part of a breaking portion 64 that breaks the sealing member 40 and a state of blood flowing through the first tube 34, is provided at an arrangement position of the first tube 34 closed by the lid body 62.

A part of the first tube 34 passing through the central region 54a1, the branching connector 32, a part of the second tube 36, and a part of the third tube 38 are disposed in a left region 54a2 (second region) of the upper surface 54a. The left region 54a2 is provided with a holder 68 that retains the branching connector 32, a PPP clamp 70 that opens and closes the second flow path 36a of the second tube 36, and a liquid drug clamp 72 that opens and closes the third flow path 38a of the third tube 38.

A part of the segment tube 28 passing through the central region 54a1 is disposed in a right region 54a3 (third region) of the upper surface 54a. The right region 54a3 is provided with a segment pocket 74 that accommodates the plurality of sealed tubes 28a of the segment tube 28.

Further, a tube retaining portion 76 protruding upward from the upper surface 54a is provided on the radially outer side of the PPP bag pocket 58 and the liquid drug bag pocket 60 of the unit set area 54. The tube retaining portion 76 has an outer wall 78 continuous with an outer peripheral edge of the unit set area 54 and an inner wall 80 protruding to the inner side of the outer wall 78 from the outer peripheral edge, and the second tube 36 is disposed between the outer wall 78 and the inner wall 80. The inner wall 80 extends from the vicinity of the PPP clamp 70 in the left region 54a2 (left side of the liquid drug bag pocket 60) to a circumferential intermediate position of the PPP bag pocket 58, and guides the second tube 36 to the PPP bag pocket 58.

In addition, the unit set area 54 includes a slider 82 (pusher), which presses the blood bag 22 after centrifugation, on the radially inner side of the blood bag pocket 56. A surface of the slider 82 facing the blood bag 22 is formed on an inclined surface 82a. The slider 82 moves forward and backward along the radial direction of the centrifugal drum 48 under the control of the control unit 50 (see Fig. 1).

In the unit set area 54, the user accommodates the blood bag 22 storing the removed blood, the empty PPP bag 24, and the liquid drug bag 26 storing the liquid drug in the blood bag pocket 56, the PPP bag pocket 58, and the liquid drug bag pocket 60, respectively. Then, the unit set area 54 centrifuges the removed blood in the blood bag 22 by the rotation of the centrifugal drum 48, advances the slider 82 after the centrifugation to press the blood bag 22. As a result, PPP generated by the centrifugation in the blood bag 22 is transported to the PPP bag 24, and RBC remains in the blood bag 22 after the transport of PPP. Thereafter, the blood bag system 10 is taken out from the centrifugal transport device 12 by the user, and the liquid drug bag 26 is hung on a stand (not illustrated). As a result, the red blood cell preservation solution is supplied from the liquid drug bag 26 to the blood bag 22, and the blood bag 22 is turned into a state of storing the RBC (blood products) containing a liquid drug.

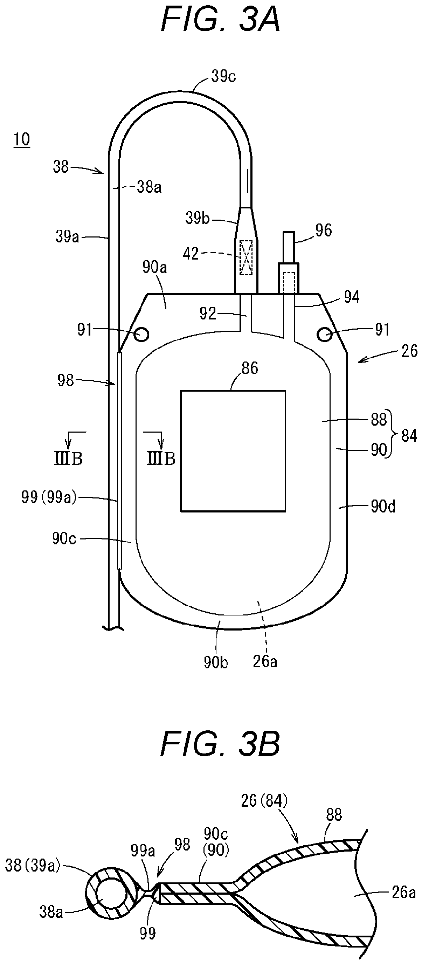

Next, the liquid drug bag 26, which is a main part of the blood bag system 10 according to the present embodiment, the third tube 38 connected to the liquid drug bag 26, and the like will be described with reference to Figs. 3A and 3B.

The liquid drug bag 26 includes a bag main body 84 having a storage space 26a capable of storing blood (removed blood). The bag main body 84 is formed in a bag shape by overlapping sheets to seal the periphery. In addition, a label 86 on which predetermined information has been printed is attached to the outer surface of the bag main body 84.

Specifically, the bag main body 84 includes an accommodating portion 88 forming the storage space 26a and a seal part 90 which is a portion sealed around the accommodating portion 88 in plan view (front view seen from the label 86 side illustrated in Fig. 3A). A resin material forming the bag main body 84 is not particularly limited, and examples thereof include polyolefins such as polyvinyl chloride, an ethylene-vinyl acetate copolymer, polyethylene, and polypropylene.

In addition, in plan view, the seal part 90 includes: a first seal part 90a which is a short side seal part forming an upper side of the bag main body 84; a second seal part 90b which is a short side seal part forming a lower side of the bag main body 84; a third seal part 90c which is a long side seal part forming a left side of the bag main body 84; and a fourth seal part 90d which is a long side seal part forming a right side of the bag main body 84. A plurality (a set) of bag holes 91 are provided in upper portions (corner portions continuous with the first seal part 90a) of the third seal part 90c and the fourth seal part 90d.

A connection port 92 to which the third tube 38 is connected and a filling port 94 to which a filling tube 96 is connected are provided on the upper side of the bag main body 84. The filling tube 96 is used when the liquid drug is stored in the storage space 26a, and is cut and sealed after being filled with the liquid drug. That is, the filling tube 96 is formed as short as possible with a sealed end portion in a state of the blood bag system 10 provided as a product.

Each of the ports 92 and 94 is welded in the state of communicating with the storage space 26a at the time of forming the seal part 90. In addition, the ports 92 and 94 are firmly fixed to end portions of the respective tubes 38 and 96 by an appropriate fixing means such as welding and adhesion. The third tube 38 includes an elongated tube main body 39a forming most of its entire length, and a cylindrical end structure 39b that is continuous with an end portion of the tube main body 39a and is formed thick to be connected to the connection port 92. The above-described sealing member 42 is provided inside the end structure 39b.

Examples of a material forming the third tube 38 include a fluorine-based resin such as polytetrafluoroethylene (PTFE), an ethylene-tetrafluoroethylene copolymer (ETFE), and a perfluoroalkoxy fluorine resin (PFA), an olefin-based resin such as polyethylene and polypropylene or a mixture thereof, polyurethane, polyester, polyamide, a polyether nylon resin, a mixture of the olefin-based resin and an ethylene-vinyl acetate copolymer, and the like.

Further, a retention mechanism 98 is provided between the liquid drug bag 26 and the third tube 38 in the blood bag system 10 according to the present embodiment. The retention mechanism 98 has a function of separably retaining the third tube 38 with respect to an outer surface of the liquid drug bag 26.

The retention mechanism 98 is provided in the third seal part 90c forming one of the long sides of the liquid drug bag 26 in the seal part 90 of the bag main body 84. The retention mechanism 98 includes a connection part 99 in which the third tube 38 and the third seal part 90c are integrally connected. The connection part 99 is configured by fusing the third tube 38 and the third seal part 90c, which have been previously molded, with a predetermined resin material. The resin material of the connection part 99 may be appropriately selected according to the material forming the third tube 38 or the third seal part 90c, and for example, the same material as that of the third tube 38 or the third seal part 90c can be applied.

The connection part 99 is provided over substantially the entire length in the extending direction of the third seal part 90c. Incidentally, an extending length of the connection part 99 is not particularly limited, but is preferably set to 50% or more of the entire length of the third seal part 90c. In addition, in a case where the entire length of the connection part 99 is short, a position where the connection part 99 is formed is preferably close to the upper side (first seal part 90a) in the third seal part 90c.

The connection part 99 connects the third tube 38 and the third seal part 90c, but is formed to be thinner than a thickness of the third tube 38 itself or a thickness of each of a plurality of sheets forming the bag main body 84. That is, an outer peripheral surface of the third tube 38 and an outer surface of the bag main body 84 are continuous by the connection part 99, but a constriction is formed at a boundary portion between the outer peripheral surface of the third tube 38 and the outer surface of the bag main body 84.

The connection part 99 forms a fragile structure 99a, which is more fragile than the third tube 38 and the bag main body 84, due to the constriction. That is, the connection part 99 is broken along the constriction based on an external force fir separating the third tube 38 and the third seal part 90c from each other. Incidentally, the fragile structure 99a is not limited to the constriction, and can adopt various structures. For example, the fragile structure 99a may be configured by applying and fusing a material brittler than the material forming the third tube 38 or the bag main body 84, or by providing a fold, a cut, a perforation, or the like in the connection part 99.

Meanwhile, the third tube 38 (tube main body 39a) has a curved portion 39c that extends from the end structure 39b in a direction orthogonal to the first seal part 90a and is curved and folded without kinking. Then, the third tube 38 is retained (connected to) in the third seal part 90c via the connection part 99 at a portion (intermediate portion between one end and the other end) extending linearly via the curved portion 39c.

The blood bag system 10 according to the present embodiment is basically configured as described above, and operations thereof will be described hereinafter.

As described above, the blood bag system 10 stores the removed blood, obtained by removing a predetermined component (white blood cell) from the whole blood of the donor, in the blood bag 22 by using the pretreatment unit at the time of collecting blood by the user (medical worker).

Thereafter, the user separates the separator unit 16 of the blood bag system 10 from the pretreatment unit and sets the separator unit 16 in the centrifugal transport device 12 in order to centrifuge the removed blood. The blood bag 22 is accommodated in the blood bag pocket 56 of the unit set area 54. In addition, the first to third tubes 34, 36, and 38 are disposed along the upper surface 54a of the unit set area 54.

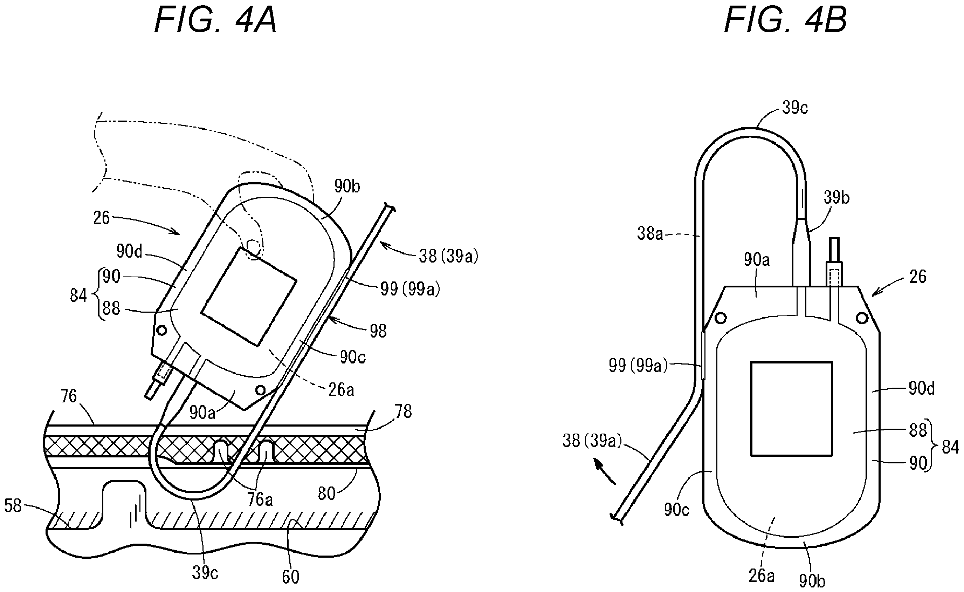

The third tube 38 extends to the radially outer side through the left region 54a2 of the upper surface 54a and is accommodated in the liquid drug bag pocket 60 together with the liquid drug bag 26. At this time, as illustrated in Fig. 4A, the user directs the first seal part 90a side downward to enter an opening of the liquid drug bag pocket 60 from the curved portion 39c of the third tube 38.

Here, the liquid drug bag 26 and the third tube 38 are integrated by the retention mechanism 98 (connection part 99). That is, the third tube 38 extends substantially linearly from the curved portion 39c along the third seal part 90c with respect to the liquid drug bag 26, and the third tube 38 is prevented from being caught by a wall portion forming the liquid drug bag pocket 60.

For example, a wall portion (inner wall 80) of the unit set area 54 forms the tube retaining portion 76 and has a plurality of protrusions 76a for guiding the accommodation of the second tube 36, but the third tube 38 is prevented from being caught by the protrusions 76a. That is, the retention mechanism 98 can restrict a free state of the third tube 38 with respect to the liquid drug bag 26 and ensure the linearity of an insertion portion of the third tube 38 with respect to the liquid drug bag pocket 60. The third tube 38 that has been inserted into the liquid drug bag pocket 60 with the liquid drug bag 26 facing downward does not greatly protrude from the liquid drug bag pocket 60, and thus, does not interfere with the periphery at the time of centrifugation.

After the blood bag system 10 is set, the centrifugal transport device 12 centrifuges the removed blood in the blood bag 22 into blood components having different specific gravities (PPP, RBC, and the like) by rotating the centrifugal drum 48 under the control of the control unit 50. After such centrifugation, the centrifugal transport device 12 presses the blood bag 22 by the slider 82. As a result, PPP having a light specific gravity flows out from the blood bag 22, and the PPP flows through the first tube 34, the branching connector 32, and the second tube 36 in this order and flows into the PPP bag 24.

When PPP is transported from the blood bag 22 to the PPP bag 24, the centrifugal transport device 12 retracts the slider 82 so that the blood bag 22 can be taken out from the blood bag pocket 56. Thereafter, the user removes the blood bag system 10 from the centrifugal transport device 12. The liquid drug bag 26 is also taken out from the liquid drug bag pocket 60 in the state of being integrated with the third tube 38 by the retention mechanism 98.

After the take-out, the user operates the third tube 38 to be separated from the liquid drug bag 26. For example, as illustrated in Fig. 4B, an appropriate external force is applied to the retention mechanism 98 so as to separate the third tube 38 from the second seal part 90b side of the liquid drug bag 26. As a result, the fragile structure 99a is easily broken along the extending direction of the third seal part 90c without damaging the liquid drug bag 26 or the third tube 38. That is, the third tube 38 is turned into the free state with respect to the liquid drug bag 26.

After the liquid drug bag 26 and the third tube 38 are broken, the user suspends the liquid drug bag 26 on the stand to supply the red blood cell preservation solution in the liquid drug bag 26 to the blood bag 22. As a result, RBC containing the red blood cell preservation solution is stored in the blood bag 22.

Incidentally, the present invention is not limited to the above-described embodiment, and various modifications can be made in accordance with a gist of the invention. For example, the third tube 38 is connected to the third seal part 90c in the seal part 90 surrounding the accommodating portion 88 in the above-described embodiment. However, the free state with respect to the liquid drug bag 26 is restricted even when the third tube 38 is connected to the seal part 90 other than the third seal part 90c, and the accommodation in the liquid drug bag pocket 60 can be facilitated.

Hereinafter, some other embodiments according to the present invention will be described. Incidentally, an element having the same configuration or the same function as that of the above-described embodiment will be denoted by the same reference sign, and the detailed description thereof will be omitted in the following description.

### [Second Embodiment]

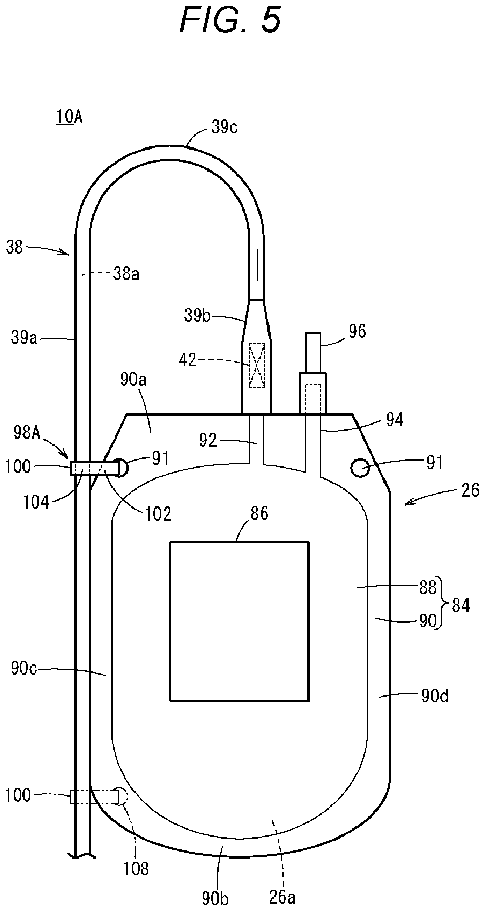

As illustrated in Fig. 5, a blood bag system 10A according to a second embodiment is different from the blood bag system 10 described above in terms of using a retaining member 100 regarding a retention mechanism 98A for the liquid drug bag 26 and the third tube 38. The retaining member 100 connects the liquid drug bag 26 and the third tube 38 in a state of the blood bag system 10A provided as a product, and separates the liquid drug bag 26 and the third tube 38 based on application of a predetermined external force.

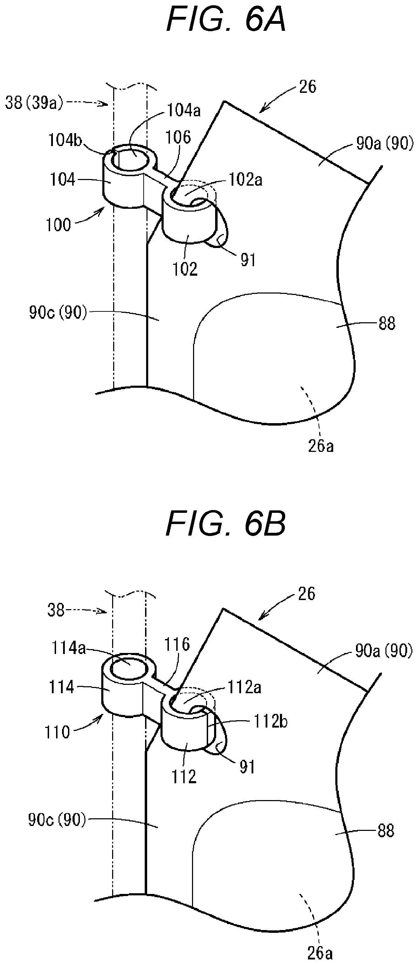

Specifically, the retaining member 100 passes through the bag hole 91 formed in the seal part 90 (corner portion where first seal part 90a and third seal part 90c intersect each other) of the liquid drug bag 26, and retains an intermediate position of the third tube 38. As illustrated in Fig. 6A, the retaining member 100 includes: a bag connecting portion 102 formed in an annular shape in plan view; a tube connecting portion 104 formed in a C shape in plan view; and a relay connecting portion 106 connected to the bag connecting portion 102 and the tube connecting portion 104.

The bag connecting portion 102 has a hole 102a having a size that allows passage of a part of the seal part 90 (a seal part on the left side of the bag hole 91). That is, the bag connecting portion 102 is undetachably connected to the liquid drug bag 26.

The tube connecting portion 104 has a hole 104a having an inner diameter substantially equal to an outer diameter of the tube main body 39a of the third tube 38. An inner surface of the tube connecting portion 104 constituting the hole 104a comes into contact with an outer peripheral surface of the third tube 38 with a certain frictional force. Therefore, the tube connecting portion 104 suppresses the movement of the third tube 38 in the axial direction.

In addition, the tube connecting portion 104 has a slit 104b communicating with the hole 104a at a position opposite to the relay connecting portion 106 with the center of the hole 104a interposed therebetween. The tube connecting portion 104 is configured such that end portions sandwiching the slit 104b can be elastically separated, and causes detachment of the third tube 38 through the slit 104b.

The retention mechanism 98A of the blood bag system 10A according to the second embodiment is configured as described above, and the retaining member 100 can separably retain the third tube 38 with respect to the liquid drug bag 26. Therefore, the blood bag system 10A can obtain the same effects as those of the blood bag system 10 according to the first embodiment. In particular, the retention mechanism 98A allows the third tube 38 to be freely attached to and detached from the liquid drug bag 26 by using the retaining member 100, and it is possible to cause the retaining member 100 to retain the third tube 38 again as necessary.

Incidentally, the retention mechanism 98A (retaining member 100) may be provided not only at one location in the liquid drug bag 26 but also at a plurality of locations. For example, another bag hole 108 may be formed in a lower portion of the third seal part 90c as indicated by a dotted line in Fig. 5, and the retaining member 100 may be provided in the bag hole 108, in addition to the retaining member 100 provided in the bag hole 91 in an upper portion of the third seal part 90c.

In addition, a retaining member 110 may include a bag connecting portion 112 separable from the liquid drug bag 26 as illustrated in Fig. 6B. For example, the bag connecting portion 112 includes a hole 112a and a notch 112b that communicates with the hole 112a and can pass through the seal part 90. Meanwhile, a tube connecting portion 114, which is connected to the bag connecting portion 112 via a relay connecting portion 116, is formed in an annular shape having a hole 114a, and can be undetachably attached to the third tube 38.

### [Third Embodiment]

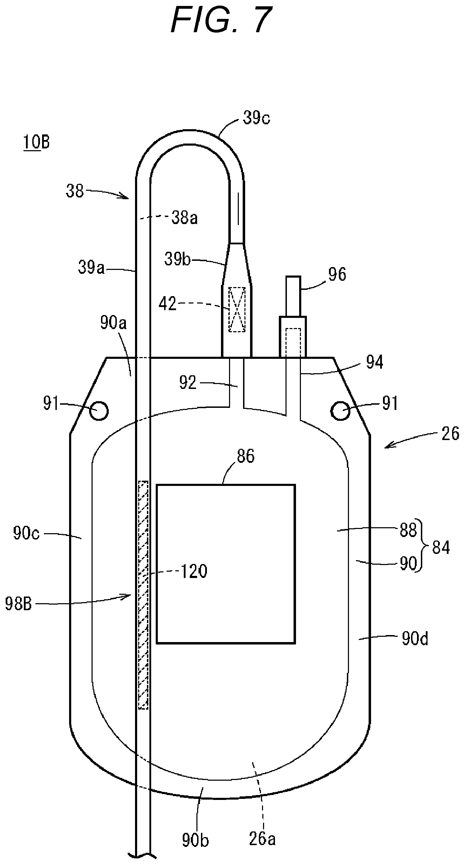

As illustrated in Fig. 7, a blood bag system 10B according to a third embodiment is different from the blood bag systems 10 and 10A described above in terms of attaching the third tube 38 to an outer surface of the liquid drug bag 26 regarding a retention mechanism 98B for the liquid drug bag 26 and the third tube 38. That is, the retention mechanism 98B has an attachment portion 120 between the liquid drug bag 26 and the third tube 38.

The attachment portion 120 is disposed on an outer surface of the accommodating portion 88 in the bag main body 84 and has adhesiveness to detachably retain the tube main body 39a of the third tube 38. The attachment portion 120 can be formed by applying an appropriate adhesive or a pressure-sensitive adhesive. Incidentally, the attachment portion 120 may be configured using adhesiveness generated on an outer surface of the bag main body 84 at the time of sterilizing the blood bag system 10B (liquid drug bag 26). Alternatively, the attachment portion 120 may attach the third tube 38 to the outer surface of the bag main body 84 by applying another member such as a tape (not illustrated). In addition, for example, the label 86 of the liquid drug bag 26 may be used as the attachment portion 120, and attached to outer surfaces of the third tube 38 and the liquid drug bag 26.

The attachment portion 120 preferably attaches the third tube 38 to the outer surface of the bag main body 84 over a predetermined length (for example, 1/2 or more of a longitudinal length of the bag main body 84).
Incidentally, a position of the attachment portion 120 with respect to the bag main body 84 (a retaining position of the third tube 38) is not particularly limited, and may be either the accommodating portion 88 or the seal part 90.

The retention mechanism 98B of the blood bag system 10B according to the third embodiment is configured as described above, and the attachment portion 120 can separably retain the third tube 38 with respect to the liquid drug bag 26. Therefore, the blood bag system 10B can obtain the same effects as those of the blood bag systems 10 and 10A. In particular, the retention mechanism 98B can be easily configured by the attachment portion 120 in the blood bag system 10B so that the manufacturing cost can be further reduced.

Technical ideas and effects that can be grasped from the above-described embodiment are described as follows.

The blood bag systems 10, 10A, and 10B respectively include retention mechanisms 98, 98A, and 98B for retaining the intermediate portion of the tube 30 (third tube 38) and the liquid drug bag 26 in a separable manner. Therefore, the liquid drug bag 26 and the third tube 38 can be integrally inserted when the liquid drug bag 26 is accommodated in the bag accommodating portion (liquid drug bag pocket 60) of the centrifugal transport device 12. As a result, the third tube 38 is prevented from interfering with the insertion of the liquid drug bag 26, and the setting work of the centrifugal transport device 12 is made efficient. In addition, after the blood bag system 10, 10A, or 10B is taken out from the centrifugal transport device 12, the liquid drug can be favorably supplied from the liquid drug bag 26 suspended on the stand thereafter by separating the third tube 38 from the liquid drug bag 26.

In addition, the liquid drug bag 26 has the seal part 90 around the storage space 26a by sealing the plurality of sheets forming the storage space 26a, and the retention mechanism 98 or 98A is provided in the seal part 90. As a result, the blood bag systems 10 and 10A retain the third tube 38 around the storage space 26a of the liquid drug bag 26, and it is possible to ensure the visibility of the liquid drug bag 26 while suppressing a kink of the third tube 38.

In addition, the retention mechanism 98 or 98A is provided in the long side seal part (third seal part 90c) forming the long side of the liquid drug bag 26 in the seal part 90, and the tube (third tube 38) extends with one end connected to the short side seal part (first seal part 90a) forming the short side of the liquid drug bag 26 in the seal part 90, and is bent and folded to reach the retention mechanism 98 or 98A. As a result, the blood bag systems 10 and 10A can retain the third tube 38 in the state of being reliably curved, and it is possible to prevent the kink of the third tube 38 while restricting the free state of the third tube 38 near the liquid drug bag 26.

In addition, the retention mechanism 98 includes the connection part 99 in which the tube 30 (third tube 38) and the seal part 90 are integrally connected, and the connection part 99 is broken based on the external force for separating the tube 30 and the seal part 90. Since the retention mechanism 98 is configured using the connection part 99 in this manner, it is possible to reliably suppress the relative displacement of the third tube 38 with respect to the liquid drug bag 26. In addition, the retention mechanism 98 can easily separate the liquid drug bag 26 and the third tube 38 with the connection part 99 as the boundary.

In addition, the connection part 99 is continuous for a predetermined length along the seal part 90 (third seal part 90c) forming the long side. As a result, the third tube 38 can be linearly extended in the liquid drug bag 26, and the third tube 38 can be easily inserted into the liquid drug bag pocket 60.

In addition, the retention mechanism 98A includes the retaining member 100 that is attached to the liquid drug bag 26 and detachably retains the tube 30 (third tube 38). The blood bag system 10A can easily retain the third tube 38 with respect to the liquid drug bag 26 by the retaining member 100. In addition, the retaining member 100 can retain the third tube 38, that has been once separated, again in the liquid drug bag 26.

In addition, the retention mechanism 98A includes the retaining member 110 that is attached to the tube 30 (third tube 38) and detachably retains the liquid drug bag 26. In this manner, it is possible to favorably retain and separate the liquid drug bag 26 and the third tube 38 even with the retaining member 110 attached to the third tube 38.

In addition, the retaining members 100 and 110 are disposed at a plurality of locations of the seal part 90 (third seal part 90c) forming one long side of the liquid drug bag 26. As a result, the third tube 38 can be linearly extended in the liquid drug bag 26, and the third tube 38 can be easily inserted into the liquid drug bag pocket 60.

In addition, the retention mechanism 98B is configured using the attachment portion 120 to which the outer surface of the liquid drug bag 26 and the outer peripheral surface of the tube 30 (third tube 38) are attached with a predetermined adhesive force. The liquid drug bag 26 and the third tube 38 can be easily integrated by the attachment portion 120 in the blood bag system 10B, and the manufacturing cost can be reduced.

## Claims

1. A blood bag system comprising:
a liquid drug bag that has an internal storage space in which a liquid drug is stored and is accommodated in a bag accommodating portion of a centrifugal transport device; and
a tube that has one end connected to the liquid drug bag and has a flow path communicating with the storage space inside,
wherein a retention mechanism for separably retaining the liquid drug bag and the tube is provided between an intermediate portion, between the one end and another end of the tube, and the liquid drug bag.

2. The blood bag system according to claim 1, wherein
the liquid drug bag includes a seal part around the storage space by sealing a plurality of sheets forming the storage space, and
the retention mechanism is provided in the seal part.

3. The blood bag system according to claim 2, wherein
the retention mechanism is provided in a long side seal part forming a long side of the liquid drug bag in the seal part, and
the tube extends with the one end connected to a short side seal part forming a short side of the liquid drug bag in the seal part, and is bent and folded to reach the retention mechanism.

4. The blood bag system according to claim 3, wherein
the retention mechanism includes a connection part in which the tube and the seal part are integrally connected, and
the connection part is broken based on an external force for separating the tube and the seal part.

5. The blood bag system according to claim 4, wherein
the connection part is continuous for a predetermined length along the seal part forming the long side.

6. The blood bag system according to any one of claims 1 to 3, wherein
the retention mechanism includes a retaining member that is attached to the liquid drug bag and detachably retains the tube.

7. The blood bag system according to any one of claims 1 to 3, wherein
the retention mechanism includes a retaining member that is attached to the tube and detachably retains the liquid drug bag.

8. The blood bag system according to claim 6 or 7, wherein
the retaining member is disposed at a plurality of positions of a seal part forming one long side of the liquid drug bag.

9. The blood bag system according to claim 1, wherein
the retention mechanism includes an attachment portion to which an outer surface of the liquid drug bag and an outer peripheral surface of the tube are attached with a predetermined adhesive force.
